Europäisches Patentamt

European Patent Office

Office européen des brevets

(11) Veröffentlichungsnummer: **0 513 421 A1**

## (12) EUROPÄISCHE PATENTANMELDUNG

(21) Anmeldenummer: **91108005.9**

(22) Anmeldetag: **17.05.91**

(51) Int. Cl.5: **F04B 43/12, A61M 5/142**

(43) Veröffentlichungstag der Anmeldung:
**19.11.92 Patentblatt 92/47**

(84) Benannte Vertragsstaaten:
**DE ES FR GB IT SE**

(71) Anmelder: **Fresenius AG**
**Gluckensteinweg 5**
**W-6380 Bad Homburg v.d.H.(DE)**

(72) Erfinder: **Polaschegg, Hans-Dietrich, Dr.**
**Grünwiesenweg 9**
**W-6370 Oberursel 4(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

(54) **Vorrichtung zur Bestimmung des behandelten Blutvolumens bei der Hämodialyse.**

(57) Die Erfindung betrifft eine Einrichtung zur Anzeige des effektiven Blutflusses und seines zeitlichen Integrals in einem extrakorporalen Kreislauf (2). Der effektive Blutfluß wird durch Multiplikation der Umdrehungszahl des Antriebs einer Blutpume (14) mit einer Pumpenkonstante und dem nominalen Querschnitt des Pumpschlauches (13) sowie mit einem Formfaktor, der die Veränderung des Querschnitts durch den Druck stromauf der Pumpe berücksichtigt, ermittelt.

Die Integration des Blutflusses wird begonnen bzw. beendet, sobald ein im extrakorporalen Kreislauf (2) stromab der Blutbehandlungseinrichtung (3) vorgesehener Sensor (18) den Übergang von wässriger Lösung zu Blut bzw. umgekehrt anzeigt und im Falle einer Hämodialysevorrichtung unterbrochen, sobald der Dialysierflüssigkeitsfluß zum Dialysator (3) unterbrochen wird.

Fig 1

Die Erfindung betrifft ein Verfahren zur Bestimmung des effektiven Flusses einer Flüssigkeit sowie Vorrichtung zur Durchführung des Verfahrens gemäß dem Oberbegriff des Anspruchs 1 bzw. Anspruchs 4.

Die Behandlung der chronischen Niereninsuffizienz durch die Hämodialyse ist ein weitverbreitetes Verfahren, dem weltweit mehr als 300.000 Patienten das Überleben verdanken.

Die damit verbundenen Kosten sind für die Öffentlichkeit beachtlich, da die Patienten mit einem erheblichen Aufwand ca. 3 x pro Woche behandelt werden müssen.

Insofern wurde daher bereits seit längerem die Frage gestellt, welche "Behandlungsdosis" notwendig bzw. hinreichend ist.

In der sogenannten "National Cooperative Dialysis Study", die in der ersten Hälfte der 80-er Jahre in den USA durchgeführt worden ist, hat man diese Frage an einer größeren Anzahl von Patienten unter gezielten Randbedingungen untersucht.

Die von Gotch und Sargent (F.A. Gotch, J.A. Sargent:
A mechanistic analysis of the National Cooperative Dialysis Study NCDS), Kidney International 28, Seite 526-534 (1985) durchgeführte Interpretation der Ergebnisse ist heute weitgehend anerkannt. Gotch und Sargent kommen zu dem Schluß, daß bei einer dreimaligen Behandlung pro Woche eine hinreichende Behandlungsqualität erzielt wird, wenn das Produkt aus Clearance x Behandlungszeit dem Ganzkörperwasservolumen des Patienten entspricht:
kT/V = 1 (k = Harnstoffclearance, T = Behandlungszeit, V = Ganzkörperwasservolumen).

In der Praxis sollen nun die Behandlungsparameter so vorgeschrieben und kontrolliert werden, daß dieses Behandlungsziel erreicht wird. Das kann beispielsweise so erfolgen, daß die Harnstoffclearenace, die mit dem vorgeschriebenen Blutfluß erzielt wird, für einen bestimmten Dialysator aus der Tabelle des Dialysator-Herstellers entnommen und das Ganzkörperwasservolumen des Patienten dadurch geteilt wird. Hieraus erhält man dann die notwendige Behandlungszeit. Das Ganzkörperwasservolumen kann dabei entweder mit Hilfe bekannter Verfahren oder näherungsweise mit 60 % des Körpergewichts angenommen werden.

Die Harnstoffclearance selbst ergibt sich bei einem bestimmten Dialysator und vorgegebenem Dialysierflüssigkeitsfluß aus dem Blutfluß. Multipliziert man den Blutfluß mit der oben errechneten Zeit, so erhält man das Blutvolumen. In guter Näherung kann man davon ausgehen, daß das Behandlungsziel erreicht wurde, wenn das so errechnete Blutvolumen durch den Dialysator gepumpt worden ist.

Nachdem die Behandlungszeit in den letzten 10 Jahren von durchschnittlich 6 auf durchschnittlich 3 Stunden verkürzt worden ist, wirken sich Einflüsse, die den Blutfluß während der Behandlungszeit temporär verringern, nunmehr relativ stärker auf die Effektivität der Dialyse aus.

Insofern soll das behandelte Blut bei der Hämodialyse möglichst genau erfaßt werden.

Das Blut wird heute nahezu ausschließlich mit Hilfe von peristaltischen Schlauchpumpen im extrakorporalen Kreislauf gefördert. Aus der DE-OS 25 35 650 läßt sich entnehmen, wie aus dem Innendurchmesser des Pumpenschlauchs und der Pumpendrehzahl die Förderrate der Pumpe errechnet und angezeigt werden kann.

Diese Förderrate kann nun über die Zeit integriert werden, wobei man als Resultat ein Blutvolumen erhält. Ein solches ist mit der Pumpe, die von der Anmelderin für die Hämodiafiltration mit ihren Hämodialysegeräten A2008D bzw. A2008E anbietet, möglich. Wird bei dieser Pumpe der Fluß unterbrochen, so wird auch die Volumenzählung unterbrochen.

Obwohl dabei bereits ein Fortschritt bei der Beurteilung der Effektivität der Hämodialyse erzielt worden ist, stimmt das ermittelte Blutvolumen immer noch nicht mit dem tatsächlich geförderten Blutvolumen überein.

Der Erfindung liegt daher die Aufgabe zugrunde, das Verfahren und die Vorrichtung der eingangs erwähnten Art so zu verbessern, daß das errechnete Flüssigkeitsvolumen im wesentlichen mit dem tatsächlich geförderten Flüssigkeitsvolumen, insbesondere dem Blutvolumen übereinstimmt.

Die Lösung der Aufgabe erfolgt durch die kennzeichnenden Merkmale des Anspruchs 1 bzw. Anspruchs 4.

Durch die Lehre der Erfindung wird die Förderrate des Schlauchs um einen bestimmten Formfaktor korrigiert, der für jede Schlauchart spezifisch ist. Bekanntlich kollabieren Schläuche von peristaltischen Pumpen unter dem Einfluß des Ansaugunterdrucks, wodurch der effektive Pumpquerschnitt und damit die Pumprate heruntergesetzt werden. Dabei ist die Verformung des Schlauchs vom Druck abhängig, d.h. das Schlauchmaterial, die Dicke der Schlauchwand, die Temperatur der zu fördernden Flüssigkeit und dergleichen bestimmen die Steifigkeit des Materials und damit auch die Verformbarkeit. Werden da nun diese Parameter in einer Tabelle in Abhängigkeit vom Druck berücksichtigt, so kann ein derart ermittelter Formfaktor zur Korrektur des Pumpvolumens herangezogen werden.

Ausgestaltungen des Verfahrens und der Vorrichtung sind in den Unteransprüchen angegeben.

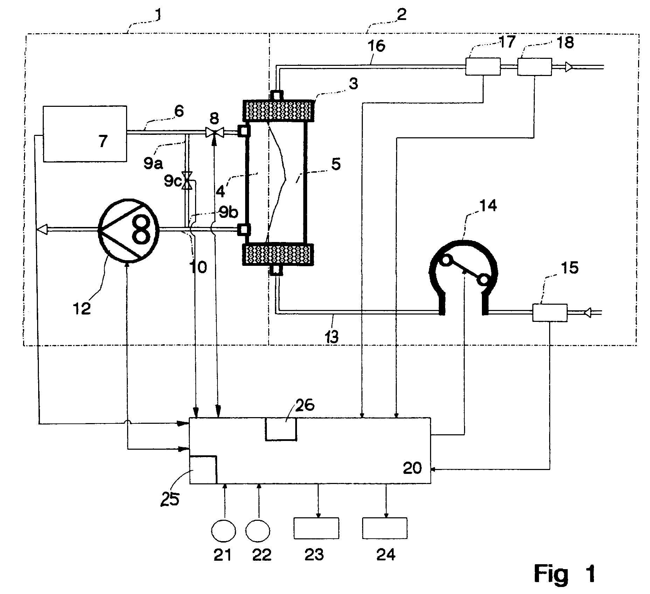

Nachfolgend wird ein Ausführungsbeispiel der Erfindung anhand der Zeichnung näher erläutert, wobei in der Figur 1 eine Hämodialysevorrichtung dargestellt ist.

Die Hömodialysevorrichtung besteht im wesentlichen aus einem Dialysierflüssigkeitsteil 1 und einem extrakorporalen Blutkreislaufteil 2, zwischen denen sich ein Dialysator 3 befindet, der durch eine semipermeable Membran 39 in eine Dialysierflüssigkeitskammer 4 und eine Blutkammer 5 geteilt ist. Die Dialysierflüssigkeitskammer 4 ist stromauf des Dialysators 3 über eine Dialysierflüssigkeitszuleitung 6 mit einer Dialysierflüssigkeitsquelle 7 verbunden. Die Zuleitung 6 weist weiterhin ein eingeschaltetes Dialysatorventil 8 auf.

Stromab des Dialysators 3 geht von der Dialysierflüssigkeitskammer 4 eine Abflußleitung 10 ab, in die eine Dialysierflüssigkeitspumpe 12 eingeschaltet ist. Zwischen der Dialysierflüssigkeitsquelle 7 und dem Dialysatorventil 8 erstreckt sich eine By-pass-Leitung 9 von der Zuleitung 6 zur Abflußleitung 10. Diese By-pass-Leitung 9 ist in die Leitungsabschnitte 9a und 9b durch das eingeschaltete By-pass-Ventil 9c geteilt.

Der extrakorporale Blutkreislauf 2 weist eine Blutzuführungsleitung 13 auf, in die eine Blutpumpe 14 eingeschaltet ist. Stromauf dieser Blutpumpe 14 ist die Blutzuführungsleitung 13 mit einem arteriellen Drucksensor 15 verbunden.

Vom Dialysator 3 geht weiterhin eine Blutabflußleitung 16 ab, in die ein venöser Drucksensor 17 und ein optischer Sensor 18 eingeschaltet sind. Weitere üblicherweise vorhandenen Einrichtungen, wie Tropfkammern und Absperrklemmen, sind in der Zeichnung nicht dargestellt.

Die Blutpumpe 14, die Dialysierflüssigkeitspumpe 12, die Dialysierflüssigkeitsquelle 7, das Dialysatorventil 8 und das By-pass-Ventil 9c sowie die Drucksensoren 15, 17 und der optische Detektor 18 sind über entsprechende elektrische Leitungen mit einer Steuereinheit 20 verbunden.

Diese Steuereinheit 20 ist fernerhin mit einer Eingabeeinheit 21, die der Eingabe von Parametern, beispielsweise des Schlauchinnendurchmessers des eingesetzten Blutschlauchs und des Schlauchtyps, dient und mit einer weiteren Eingabeeinheit 22 verbunden, mit der die Pumprate der Blutpumpe vorbestimmt werden kann. Die Steuereinheit 20 verfügt weiterhin über Anzeigeeinheiten 23 und 24, mit denen der effektive Blutfluß oder das kumulierte Blutvolumen angezeigt werden können.

Die Eingabeeinheit 21 kann beispielsweise eine Schaltereinrichtung aufweisen, die die Wahl eines bestimmten Schlauchtyps ermöglicht, wobei gleichzeitig der Innendurchmesser und das Complianceverhalten, also die Veränderung des Querschnitts und der Einfluß des anliegenden Drucks bestimmbar sind. Andererseits können jedoch auch anstelle einer solchen Eingabeeinheit 21 ein Barcodeleser oder eine andere Vorrichtung eingesetzt werden, die eine mit dem Schlauch verbundene Markierung erkennt, die in die Steuereinheit 20 eingegeben und dort weiterverarbeitet wird.

Die Eingabeeinheit 21 kann auch als eine Vorrichtung ausgebildet sein, die den Schlauchinnendurchmesser ermittelt. Eine solche Vorrichtung ist in der DE-OS 38 42 404 beschrieben, auf deren Offenbarung Bezug genommen wird. Gemäß dieser bekannten Vorrichtung wird mit Hilfe einer Kraft-Weg-Meßeinrichtung der Schlauchinnendurchmesser bestimmt, wobei sich hieraus ein der Nachgiebigkeit des Schlauchs proportionaler Parameter ableiten läßt.

Anstelle einer manuellen Einstellung des Blutflusses über die Eingabeeinheit 22 kann auch eine selbsttätige Einstellung, beispielsweise als Funktion des Drucks am Drucksensor 15 erfolgen. Die Eingabeeinheit kann dann zur Vorgabe des Solldrucks verwendet werden. Eine solche Drucksteuerung der Blutpumpe ist beispielsweise in der EP 0154681 beschrieben, auf die Bezug genommen wird.

Die Steuereinheit 20 weist ferner eine Speichereinheit 25 auf, in die Daten über die Querschnittsveränderung unterschiedlicher Schlauchtypen unter Einfluß des stromauf anliegenden Drucks gespeichert sind bzw. speicherbar sind. Diese Daten können entweder in Form einer Tabelle oder einer Gleichung vorliegen. Diese Tabelle bzw. Gleichung ergibt einen Formfaktor, dessen Wert beim Druck null 1 ist und dessen Wert für Unterdrucke kleiner 1 und für Überdrucke größer 1 wird. Dieser Formfaktor ist ebenfalls zu den Schlauchparametern zu rechnen. Ein typischer Wert für einen Unterdruck von minus 100 mmHg ist beispielsweise 0,9 für einen typischen Schlauch aus Siliconmaterial mit einem nominalen Innendurchmesser von 8 mm, d.h. der Querschnitt verändert sich um den Faktor 0,9 bei diesem Unterdruck, was zur Folge hat, daß 10 % weniger Flüssigkeit durch den Schlauch gefördert werden.

Die Ermittlung des effektiven Blutflusses für eine rotierende peristaltische Pumpe erfolgt durch die Multiplikation der Umdrehungsgeschwindigkeit der Pumpe (U/min) mit der Pumpkonstante, die dem Umfang der Pumpschlauchachse entspricht (cm/U), dem Nominalquerschnitt des Schlauchs, der aus dem Innendurchmesser berechnet werden kann ($cm^2$) und dem oben erwähnten dimensionslosen Formfaktor, der die Druckabhängigkeit berücksichtigt. Das Produkt der oben angegebenen Dimensionen ergibt folgerichtig $cm^3$/min.

Dieser effektive Blutfluß läßt sich durch eine weitere Einrichtung über die Zeit integrieren. Die Integration beginnt in jenem Moment, da der optische Sensor 18 den Farbumschlag von hell auf dunkel registriert, womit angezeigt wird, daß nunmehr Blut behandelt wird, während zuvor das Schlauchsystem in bekannter Weise lediglich mit physiologischer Kochsalzlösung vorgefüllt war. Die Integration wird immer dann unterbrochen, wenn das By-pass-Ventil 9c geöffnet oder das Dialysatorventil 8 geschlossen ist. Dies erfolgt in bekannter Weise immer dann, wenn die Dialysierflüssigkeit eine falsche Zusammensetzung oder eine falsche Temperatur besitzt. Andererseits kann auch das Dialysatorventil 8 zur periodischen Rekalibrierung oder Prüfung von Einrichtungen des Dialysierflüssigkeitssystems geschlossen werden.

In einer weiteren Ausgestaltung der Erfindung kann die oben erwähnte Eingabeeinrichtung 21 oder eine weitere Eingabeeinrichtung dazu dienen, den Dialysatortyp, das Patientengewicht oder das Patientenkennzeichen zu erfassen und einzugeben.

In einer weiteren Speichereinrichtung 26 wird vorteilhaft das Datenfeld Clearance/Blutfluß und der Dialysierflüssigkeitsfluß für verschiedene Dialysatortypen gespeichert. Darüber hinaus kann eine zusätzliche Speichereinrichtung auch noch das, zu einem bestimmten Patientenkennzeichen (Name, Nummer) gehörende Ganzkörperwasser oder Gewicht enthalten.

Aus dem effektiven Blutfluß, dem Dialysierflüssigkeitsfluß (der durch die Verbindung der Pumpe 12 mit der Steuer- und Auswerteeinheit 20 erfaßbar ist) und dem Dialysatordatenfeld läßt sich somit die Clearance errechnen und diese über die Zeit integrieren, womit man k.T erhält. Wird zusätzlich, wie erwähnt, daß Patientengewicht bzw. das Patientenganzkörperwasser erfaßt, so läßt sich die Größe kT/V errechnen und über eine der erwähnten Anzeigevorrichtungen 23,24 darstellen.

Mit Hilfe des Verlaufs dieser Größen und durch Vergleich mit einem vorgegebenen Sollwert läßt sich beispielsweise das Dialyseende vorausschauend hochrechnen oder das Erreichen des Sollwerts durch ein geeignetes Signal angeben.

Aus der DE-OS 39 38 662 ist die Bestimmung der Harnstoffclearance während der Hämodialyse mit Hilfe einer Leitfähigkeitsrelativmessung bekannt. Diese Bestimmung läß sich mit der vorliegenden Anordnung gemäß Figur 1 folgendermaßen kombinieren. Zu Beginn der Hämodialyse wird die Clearance bestimmt und das Resultat zur Korrektur des gespeicherten Clearance-Leistungsfelds (Clearance gegen Blut- und Dialysierflüssigkeitsfluß) bzw. der gespeicherten Clearanceformel verwendet. Die kumulierte Größe k.T wird dann erfindungsgemäß, wie weiter oben beschrieben, bestimmt.

Im Laufe der Dialyse (beispielsweise nach einer Stunde) wird die Clearancemessung wiederholt und das Resultat mit dem Wert aus dem korrigierten Clearanceleistungsfeld verglichen. Bei einer Differenz, die größer als die Meßtoleranz (typisch +/- 5 %) ist, wird das Clearanceleistungsfeld korrigiert und von da an mit dem neuen Clearancewert integriert.

Üblicherweise sind peristaltisch arbeitende Pumpen so ausgelegt, daß die Pumprate im Arbeitsbereich unabhängig vom Druck stromab der Pumpe ist. Sollte im Ausnahmefall doch eine Abhängigkeit der Pumprate vom Druck stromab der Pumpe bei einem bestimmten Pumptyp vorliegen, so läßt sich die erfindungsgemäße Einrichtung ebenfalls auf diesen Fall anwenden. Dabei wird ein weiterer Drucksensor stromab der Blutpumpe 14 vorgesehen, wobei der Faktor dann über ein zweidimensionales Datenfeld (Druck stromauf, Druck stromab) ermittelt wird.

Andererseits ist die Erfindung nicht auf rotierende, peristaltisch arbeitende Pumpen beschränkt, sondern vielmehr unangepaßt auch auf andere Pumpen anwendbar, wenn deren Förderrate vom Druck abhängt. Lediglich die oben erwähnte Pumpenkonstante (Nominalrate/Undrehungszahl) muß dann der Pumpenkonstruktion entsprechend angepaßt werden.

Die Erfindung ist ferner nicht auf Dialysevorrichtungen beschränkt, sondern vielmehr bei allen Blutbehandlungseinrichtungen anwendbar, bei denen ein Zusammenhang zwischen gepumpter Blutmenge und Behandlungseffektivität besteht. Dazu gehören z.B. Einrichtungen zur Bestrahlung von Blut mit Licht. Die Unterbrechung der Integration des Blutflusses erfolgt dann nicht, wie bei der Unterbrechung des Dialysierflüssigkeitsflusses, durch eine By-pass-Leitung, sondern - der Behandlungsmethode entsprechend - durch Unterbrechung der Lichtbestrahlung.

Grundsätzlich ist die erfindungsgemäße Vorrichtung auch für die Hämo- oder Plasmafiltration geeignet, wobei jedoch bei diesen Behandlungen vorteilhafterweise die durch Filtration ausgetauschte Menge zur Beurteilung der Behandlungseffektivität herangezogen wird.

Bei der Hämodiafiltration (Kombination von Hämofiltration und Hämodialyse) ist schließlich eine Kombination der beiden Beurteilungsverfahren (Blutvolumen und Austauschvolumen) vorteilhaft.

**Patentansprüche**

1. Verfahren zur Bestimmung des effektiven Flusses einer Flüssigkeit, die in einem elastischen Schlauch durch die Wirkung einer peristaltischen Pumpe gefördert wird, bei dem man die Pumprate mit der Pumpkonstante der peristaltischen Pumpe und dem Schlauchinnenquerschnitt multipliziert, dadurch gekennzeichnet, daß der nominale Schlauchinnenquerschnitt mit einem druckabhängigen Formfaktor multipliziert wird.

2. Verfahren nach Anspruch 1, dadurch gekennzeichnet, daß man den Innenquerschnitt des elastischen Schlauchs bei verschiedenen Drükken bestimmt und hieraus gegenüber dem Innendurchmesser eines unbelasteten Schlauchs den Formfaktor ermittelt, die Vielzahl von Formfaktoren in einer Tabelle ablegt und die Tabellenwerte in Abhängigkeit vom jeweils ermittelten Druck zur Bestimmung des aktuellen Innendurchmessers heranzieht.

3. Verfahren nach Anspruch 1 oder 2, dadurch gekennzeichnet, daß man den effektiven Fluß über die Zeit integriert.

4. Vorrichtung zur Bestimmung des effektiven Flusses einer Flüssigkeit, insbesondere von Blut in einem elastischen Schlauch (13), der in einer peristaltischen Pumpe (14) angeordnet ist, mit einem Drucksensor (15) stromauf dieser Pumpe (14) sowie einer Betriebseinheit (20), dadurch gekennzeichnet, daß die Betriebseinheit (20) eine Recheneinheit aufweist, die die Pumprate, mit der die Flüssigkeit gefördert wird, mit der Pumpkonstante und dem nominalen Schlauchinnenquerschnitt unter Erhalt der nominalen Förderrate/Zeiteinheit multipliziert und diesen erhaltenen Wert mit einem Formfaktor korrigiert, der durch die Veränderung des Schlauchquerschnitts durch den stromauf der Pumpe anliegenden Druck bestimmt ist.

5. Vorrichtung nach Anspruch 4, dadurch gekennzeichnet, daß die Betriebseinheit (20) eine Speichereinheit (25) aufweist, in der Formfaktoren unterschiedlicher Schlauchmaterialien gespeichert sind.

6. Vorrichtung nach Anspruch 4 oder 5, gezeichnet durch eine Integrationseinrichtung, die den effektiven Blutfluß über die Zeit integriert.

7. Vorrichtung nach Anspruch 6, dadurch gekennzeichnet, daß die Betriebseinheit (20) die Integrationseinrichtung bei geschlossenem Bypass-Ventil (9), geöffnetem Dialysatorventil (8) oder einem vorbestimmten Signalwert eines optischen Detektors (18), der an der Leitung angeordnet ist, aktiviert.

8. Vorrichtung nach einem der Ansprüche 4-7, dadurch gekennzeichnet, daß eine Eingabeeinheit (21) zur Fassung eines Dialysatortyps und eine zweite Speichereinheit (26) zur Speicherung des Clearancedatenfeldes unterschiedlicher Dialysatortypen (Clearance gegen Blut- und Dialysierflüssigkeitsfluß) und eine weitere Anzeigeeinheit zur Anzeige der momentanen Clearance sowie der akkumulierten Clearance (k x T) vorgesehen sind.

9. Vorrichtung nach Anspruch 8, dadurch gekennzeichnet, daß eine Eingabe- und Recheneinheit zur Erfassung und/oder Berechnung des Patientenganzkörperwassers (V) und eine Anzeigeeinrichtung zur Anzeige der Größe k x T/V vorgesehen sind.

10. Vorrichtung nach Anspruch 9, gekennzeichnet durch eine weitere Recheneinheit, die durch Vergleich von Soll- und aktuellem Ist-Wert für die Größe k x T/V die voraussichtlich noch notwendige Dialysezeit hochrechnet und über eine Anzeigeeinheit anzeigt.

Fig 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 393 354 (MOSEBACH)<br>* das ganze Dokument *<br>--- | 1 | F04B43/12<br>A61M5/142 |
| A | WO-A-9 109 229 (D'SILVA)<br>* das ganze Dokument *<br>--- | 1 | |
| A | DE-A-3 842 404 (FRESENIUS AG)<br>* das ganze Dokument *<br>----- | 1 | |

|  |  |  | RECHERCHIERTE SACHGEBIETE (Int. Cl.5) |
|---|---|---|---|
|  |  |  | F04B<br>A61M |

Der vorliegende Recherchenbericht wurde für alle Patentansprüche erstellt

| Recherchenort | Abschlußdatum der Recherche | Prüfer |
|---|---|---|
| DEN HAAG | 26 FEBRUAR 1992 | VON ARX H.P. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
...............................................................
& : Mitglied der gleichen Patentfamilie, übereinstimmendes
   Dokument

EPO FORM 1503 03.82 (P0403)